(19) Europäisches Patentamt
European Patent Office
Office européen des brevets

(11) Numéro de publication: **0 040 140**
**A1**

(12) # DEMANDE DE BREVET EUROPEEN

(21) Numéro de dépôt: 81400713.4

(22) Date de dépôt: 06.05.81

(51) Int. Cl.[3]: C 07 C 69/92
C 07 C 67/31

(30) Priorité: 14.05.80 US 149588

(43) Date de publication de la demande:
18.11.81 Bulletin 81/46

(84) Etats contractants désignés:
AT BE CH DE FR GB IT LI LU NL SE

(71) Demandeur: RHONE-POULENC INDUSTRIES
22, avenue Montaigne
F-75008 Paris(FR)

(72) Inventeur: Gradeff, Peter S.
P.O. Box 278
Pottersville N.J. 07979(US)

(72) Inventeur: Schreiber, Fred G.
100 Laurence Avenue
Highland Park N.J. 08904(US)

(74) Mandataire: Cazes, Jean-Marie et al,
RHONE POULENC Service Brevets Chimie et Polymères
B.P. 753
F-75360 Paris Cedex 08(FR)

(54) **Procédé de préparation d'acides métaphénoxybenzoïques et de leurs esters alkyliques.**

(57) L'invention a pour objet un procédé de préparation d'acides métaphénoxybenzoïques et de leurs esters alkyliques, caractérisé en ce qu'il comprend la réaction d'un phénate alcalin avec un ester alkylique d'un acide métahalogénobenzoïque en présence de sulfate de cuivre comme catalyseur.

EP 0 040 140 A1

Croydon Printing Company Ltd.

PROCEDE DE PREPARATION D'ACIDES METAPHENOXYBENZOIQUES ET DE LEURS ESTERS ALKYLIQUES

La présente invention a pour objet un procédé de préparation d'acides métaphénoxybenzoïques et de leurs esters alkyliques.

Plus particulièrement, l'invention concerne la réaction d'un ester alkylique d'un acide métahalogénobenzoïque avec un sel alcalin du phénol ou d'un phénol substitué.

Cette réaction est connue dans l'art antérieur sous l'appellation générale de réaction d'Ullmann.

On connaît en particulier J. Pharm. Soc. Japan. 74, 1061 (1964) et J. Pharm. Soc. Japan. 74, 1728 (1954) qui décrivent la préparation de métaphénoxybenzoate de méthyle par la réaction d'Ullmann. Dans ces deux références, le dérivé méta-bromé est mis en oeuvre en présence d'un catalyseur constitué de cuivre et d'acétate cuivrique. Dans les deux cas, les rendements obtenus sont assez faibles, respectivement 39 et 22 %.

Le principal objet de la présente invention est de fournir un procédé pour la préparation d'acides métaphénoxybenzoïques ou de leurs esters alkyliques, selon la réaction d'Ullmann en présence d'un catalyseur permettant d'opérer dans des conditions plus douces et d'obtenir de meilleurs rendements.

La présente invention a donc pour objet un procédé de préparation d'acides métaphénoxybenzoïques et de leurs esters alkyliques, caractérisé en ce qu'il comprend la réaction d'un phénate alcalin avec un ester alkylique d'un acide métahalogénobenzoïque en présence de sulfate de cuivre comme catalyseur.

Le procédé selon l'invention peut être représenté par la réaction suivante dans laquelle A représente l'ester ci-dessus et B le phénate alcalin précité :

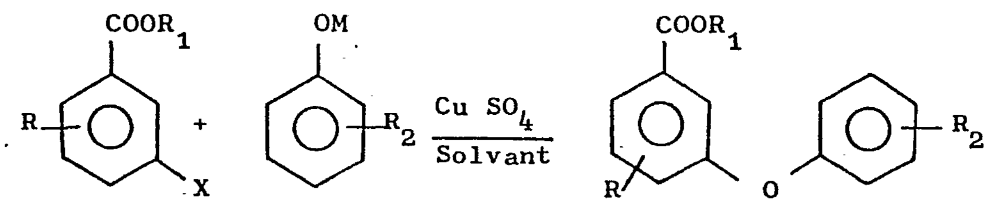


A B

où

X représente un atome d'halogène (Cl, Br, I, F),

R représente un atome d'hydrogène, un radical alkyle, cyclo-alkyle, aryle ou le radical $NO_2$,

$R_1$ représente un groupement alkyle ayant de 1 à 6 atomes de carbone, comme par exemple, les radicaux méthyle, éthyle, propyle, isopropyle, butyle, isobutyle et pentyle,

$R_2$ représente un atome d'hydrogène, au moins un radical alkyle ayant de 1 à 6 atomes de carbone, un radical alkoxy ayant de 1 à 6 atomes de carbone, un atome d'halogène ou le radical $CF_3$.

M représente Na ou K.

On peut citer comme exemples de composés B pouvant être mis en oeuvre dans le cadre du procédé selon l'invention, les composés de formules :

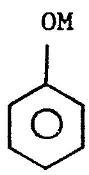 , 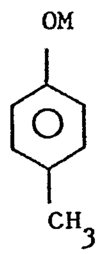 , 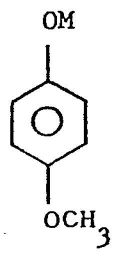 , 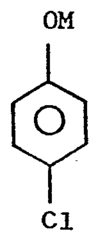 , 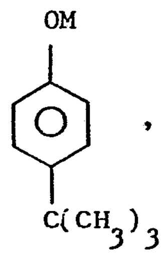 , 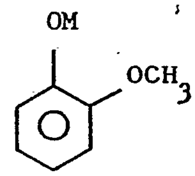

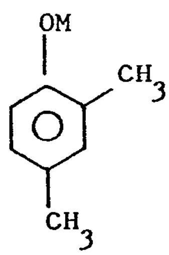 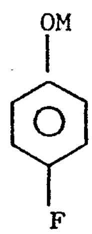 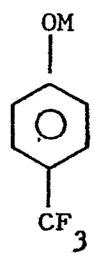

où M a la signification précédente.

Du sulfate de cuivre hydraté commercial peut être utilisé dans le cadre de l'invention. Cependant, les meilleurs résultats sont obtenus avec le sulfate de cuivre anhydre.

Le sulfate de cuivre anhydre peut être obtenu par séchage du sulfate de cuivre hydraté à température suffisamment haute pour éliminer l'eau.

On opère, de préférence, avec une quantité de sulfate de cuivre comprise entre environ 0,01 mole et environ 0,6 mole par mole d'ester alkylique de l'acide métahalogénobenzoïque. Encore plus préférentiellement, cette quantité est comprise entre environ 0,1 et environ 0,3 mole.

Le procédé selon l'invention est de préférence mis en oeuvre

BAD ORIGINAL

avec des quantités molaires équivalentes de composés A et B.

On peut utiliser tout solvant inerte dans les conditions de la réaction. Le solvant permet la mise en mélange du phénate qui est solide.

La réaction est de préférence conduite à pression atmosphérique bien que des pressions supérieures à la pression atmosphérique ne soient pas exclues du domaine de l'invention.

La température de réaction est généralement comprise entre environ 100°C et 240°C et de préférence entre 140°C et 120°C. Lorsque des températures plus faibles sont utilisées, la réaction est assez lente. Lorsque des températures plus hautes sont utilisées, la réaction est plus rapide mais la sélectivité diminue.

Les temps de réaction sont généralement compris entre environ 4 et environ 24 heures et plus particulièrement entre 6 et 16 h.

Selon une manière pratique de mettre en oeuvre le procédé de l'invention, le sulfate de cuivre et l'ester alkylique de l'acide métahalogénobenzoïque sont ajoutés au phénate et au solvant, à une température voisine de 140°C. On augmente ensuite la température jusqu'à 160 - 180°C et on maintient cette température pendant le temps de la réaction.

Le phénate mis en oeuvre dans le cadre la présente invention peut être préparé séparément ou dans le réacteur où aura lieu la réaction selon l'invention. La préparation peut se faire par action de sodium ou de potassium métallique sur le phénol correspondant ou par action d'hydroxyde correspondant avec élimination azéotropique de l'eau formée. Ceci peut être fait dans un excès de phénol ou dans un solvant comme le xylène qui servira ensuite de solvant dans la réaction de l'invention.

Le traitement du mélange réactionnel résultant dépend du produit recherché. Une hydrolyse acide ou basique permettra d'obtenir l'acide. Une estérification permettra d'obtenir l'ester.

L'invention va être plus complètement décrite à l'aide des exemples qui vont suivre :

EXEMPLE 1

On prépare 1,08 mole de phénate de sodium à partir de phénol et de sodium métallique dans 300 ml de xylène au reflux. On distille 100 ml de xylène et on ajoute 17,8 g de sulfate cuivrique Cu $SO_4$ et

BAD ORIGINAL

170,6 g de m-chlorobenzoate de méthyle. On distille une quantité supplémentaire de xylène jusqu'à ce que la température atteigne 165°C et on laisse au reflux une nuit. Le mélange réactionnel est refroidi, estérifié avec du diméthylsulfate et de nouveau, laissé au reflux une nuit. Le mélange résultant est refroidi, dilué avec de l'eau et séché. La couche organique est lavée avec de l'eau, séchée, filtrée et concentrée. Le produit brut est soumis à une distillation-flash pour donner du métaphénoxybenzoate de méthyle.

Le rendement par rapport à la quantité chargée est de 67,5 %, le rendement par rapport à la quantité transformée étant de 86,2 %.

EXEMPLE 2

On prépare 0,156 mole de phénate à partir de 16 g de phénol et 3,6 g de sodium dans du xylène sous argon. On ajoute 2,5 g de sulfate de cuivre et 30 g de m-chlorobenzoate d'isopropyle. On distille du xylène jusqu'à ce que la température du mélange atteigne 150°C. On maintient ensuite la température à 170°C pendant 21 h. On refroidit le mélange et l'on estérifie avec 5 ml de sulfate de diméthyle dans 100 ml d'acétone et on laisse au reflux une nuit.

On refroidit le mélange réactionnel, on le filtre et on le concentre par évaporation. Le solide obtenu est dissous dans l'eau, acidifié et on extrait la couche aqueuse avec du chlorure de méthylène. Cet extrait est constitué avec le concentrat et on lave le tout avec de l'eau. Après séchage, la solution est concentrée. L'huile brut est flashée sous un vide de 0,05 mm de Hg. On obtient du métaphénoxybenzoate d'isopropyle avec un rendement de 69,2 % par rapport à la quantité changée et de 83,4 % par rapport à la quantité transformée.

BAD ORIGINAL

REVENDICATIONS

1 - Procédé de préparation d'acides métaphénoxybenzoïques et de leurs esters alkyliques, caractérisé en ce qu'il comprend la réaction d'un phénate alcalin avec un ester alkylique d'un acide métahalogénobenzoïque en présence de sulfate de cuivre comme catalyseur.

2 - Procédé selon la revendication 1, caractérisé en ce que l'on met en oeuvre du sulfate de cuivre anhydre.

3 - Procédé selon l'une quelconque des revendications précédentes, caractérisé en ce que la quantité de sulfate de cuivre est comprise entre 0,01 mole et 0,6 mole par mole d'ester alkylique de l'acide métahalogénobenzoïque.

4 - Procédé selon la revendications 3 caractérisé en ce que la quantité de sulfate de cuivre est comprise entre 0,1 et 0,3 mole.

5 - Procédé selon l'une quelconque des revendications précédentes, caractérisé en ce qu'on opère à une température comprise entre 100 et 240°C.

6 - Procédé selon la revendication 5 caractérisé en ce que la température est comprise entre 140°C et 200°C.

7 - Procédé selon l'une quelconque des revendications précédentes, caractérisé en ce qu'on ajoute le sulfate de cuivre et d'ester alkylique de l'acide métahalogénobenzoïque au phénate alcalin en solution dans un solvant inerte.

8 - Procédé selon l'une quelconque des revendications précédentes caractérisé en ce que l'on fait réagir le phénate de sodium et le métachlorobenzoate de méthyle.

BAD ORIGINAL

9 - Procédé selon l'une quelconque des revendications 1 à 7, caractérisé en ce que l'on fait réagir le phénate de sodium et le métachlorobenzoate d'isopropyle.

BAD ORIGINAL

Office européen des brevets

# RAPPORT DE RECHERCHE EUROPEENNE

0040140

Numéro de la demande

EP 81 40 0713

| DOCUMENTS CONSIDERES COMME PERTINENTS | | | CLASSEMENT DE LA DEMANDE (Int. Cl.3) |
|---|---|---|---|
| Catégorie | Citation du document avec indication, en cas de besoin, des parties pertinentes | Revendication concernée | |
| X | JOURNAL OF THE CHEMICAL SOCIETY, Perkin Transactions II, 1974, Londres, GB<br>T.D. TUONG et al.: "Mechanism of the Ullmann condensation reaction. Part III. Role of the copper catalyst"<br>* En entier *<br>-- | 1-9 | C 07 C 69/92<br>67/31 |
| X | CHEMICAL ABSTRACTS, vol. 80, no. 1 07-01-1974, page 282, abrégé no. 3250u<br>Columbus, Ohio, US<br>& JP - A - 73 61443 (SUMITOMO CHEMICAL CO.)(28-08-1973)<br>* En entier *<br>-- | 1-9 | DOMAINES TECHNIQUES RECHERCHES (Int. Cl.3)<br>C 07 C 69/92<br>67/31 |
| X | CHEMICAL ABSTRACTS, vol. 49, no. 21, 10-11-1955, colonne 14683b<br>Columbus, Ohio, US<br>M. TOMITA et al.: "Synthesis of hydroxamic acid derivatives containing diphenyl ether nucleus"<br>& J. Pharm. Soc. Japan, 74, 1278-9 (1954)<br>* En entier *<br>-- | 1-9 | CATEGORIE DES DOCUMENTS CITES<br>X: particulièrement pertinent<br>A: arrière-plan technologique<br>O: divulgation non-écrite<br>P: document intercalaire<br>T: théorie ou principe à la base de l'invention<br>E: demande faisant interférence<br>D: document cité dans la demande<br>L: document cité pour d'autres raisons |
| | FR - A - 2 392 950 (CRODA SYNTHETIC CHEMICALS LTD)<br>* En entier *<br>---- | 1-9 | &: membre de la même famille, document correspondant |
| X | Le présent rapport de recherche a été établi pour toutes les revendications | | |

| Lieu de la recherche | Date d'achèvement de la recherche | Examinateur |
|---|---|---|
| La Haye | 06-08-1981 | ALLARD |

OEB Form 1503.1 06.78